# EUROPEAN PATENT APPLICATION

(11) **EP 2 716 255 A1**
(43) Date of publication of application: **09.04.2014**
(21) Application number: 12792879.4
(22) Date of filing: 27.04.2012
(51) Int. Cl.: A61C 8/00, A61C 3/02

(54) **DENTAL IMPLANT JIG, SET THEREOF, DRILLING BAR, AND SET THEREOF**

(30) Priority: 01.06.2011 JP 2011123461; 25.04.2012 JP 2012099437
(71) Applicant: Eintellex Co., Ltd., Osaka 534-0025 (JP)
(72) Inventor: ENOMOTO Mamiko, Sakai-shi Osaka 592-8344 (JP); ENOMOTO Akifumi, Osakasayama-shi Osaka 589-8511 (JP)
(74) Representative: TBK
(86) International application number: PCT/JP2012/061366
(87) International publication number: WO 2012/165093

(57) **Abstract**

The present invention provides a dental implant jig, a dental implant jig set, a drill bar, and a drill bar set, which can safely and easily achieve accurate drilling at low costs. The dental implant jig according to the present invention is provided at a drill bar of a dental hand piece to act as a guide in position and/or direction of drilling for forming an implant fixture embedding hole, is integrated with a shaft interposed between an attachment portion on one side of the drill bar, which is attached to the hand piece, and a blade portion on the other side of the drill bar, rotates around the center of the shaft together with the shaft, and forms a guide portion that is provided on the outer side of the shaft concentrically with the shaft and has a larger diameter than the shaft.

## Description

### TECHNICAL FIELD

The present invention relates to a dental implant jig, a dental implant jig set, a drill bar and a drill bar set, which are suitable for a drilling operation in dental implant treatment.

### BACKGROUND ART

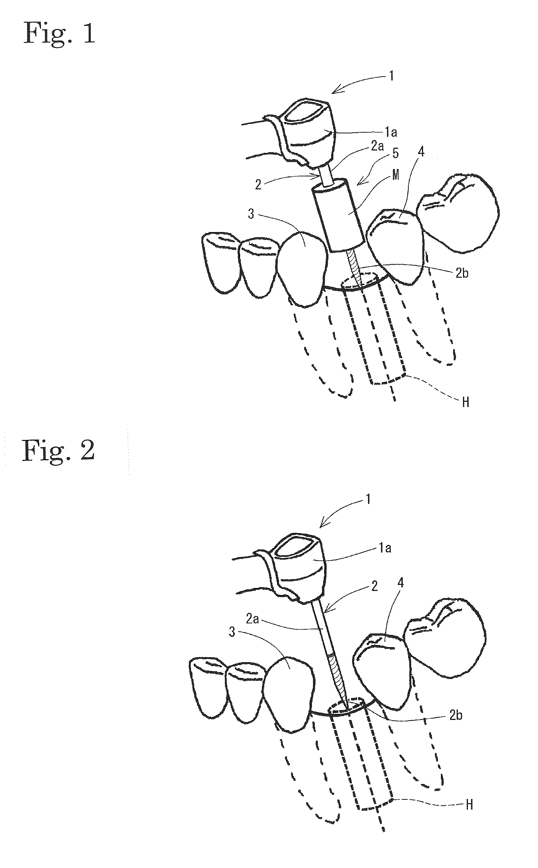

In the dental implant treatment, it is important to embed an implant fixture at an ideal position, and the success or the failure of embedding largely depends on the performance of drilling for forming a fixture embedding hole. The drilling may be performed freehand by an operator with a dental hand piece 1 having a head 1a attached to a drill bar 2 (Refer to Fig. 2) and however, the drilling cannot be reattempted and thus, various jigs has been proposed to improve the correctness of the drilling.

A typical jig is a resin stent that is produced based on an impression of a chipped portion of a patient's tooth, and is used to cover a teeth row including the chipped portion (Refer to Patent Document 1, for example). This type of stent has a guide hole for guiding the drill bar 2 to a proper drilling position.

### PRIOR ART DOCUMENT

### PATENT DOCUMENT

Patent Document 1: Japanese Unexamined Patent Publication No. 2003-245289

### SUMMARY OF THE INVENTION

### PROBLEMS TO BE SOLVED BY THE INVENTION

However, such stent has following disadvantages. First, since a play (clearance) is provided between the guide hole of the stent and the drill bar so as not to bring the drill bar into contact with the stent, the entry angle of the drill bar 2 may be tilted relative to the guide hole due to the play, resulting in drilling of a site displaced from a planned position. Moreover, for the partial cover-typed stent, the stent itself covering the teeth row is easy to rattle, and such rattle can lead to the failure of the drilling. For the full cover-typed stent, a rattle hardly occurs, but the stent masks most of the surgical site, making it difficult to find possible entry of the drill bar 2 to an improper position. Accordingly, use of the stent cannot necessarily achieve accurate drilling. Moreover, when the drill bar 2 contacts the stent by mistake and cuts a part of the stent, cuttings can enter into the fixture embedding hole, causing inflammation around the implant or obstructing osseointegration (bone bonding).

The drilling is often performed freehand without using the stent that requires high manufacturing costs as being unreasonable especially in implant prosthesis for partial chipping and a few teeth chipping. In this case, the operator creates an image H of an ideal embedding hole and an image of an upper prosthesis by using a shaft 2a of the drill bar 2 and teeth 3 and 4 positioned at both sides of the chipped portion as guides, and decides the position and the direction of drilling on the basis of only the images (Refer to Fig. 2). However, the specified diameter of the shaft 2a is generally, about only 2 mm, and the narrowness is inconvenient in creating the images and visually deciding a starting point, contributing to displacement of drilling in position and direction.

The present invention is conceived in consideration of the above-mentioned situation, and its object is to provide a dental implant jig, a dental implant jig set, a drill bar, and a drill bar set, which can safely and easily achieve accurate drilling at low costs.

### SOLUTIONS TO THE PROBLEMS

To attain the above-mentioned object, a dental implant jig according to the present invention is provided at a drill bar of a dental hand piece to act as a guide in position and/or direction of drilling for forming an implant fixture embedding hole, is integrated with a shaft interposed between an attachment portion on one side of the drill bar, which is attached to the hand piece, and a blade portion on the other side of the drill bar, rotates around the center of the shaft together with the shaft, and forms a guide portion that is provided on the outer side of the shaft concentrically with the shaft and has a larger diameter than the shaft (claim 1).

In the dental implant jig, the guide portion may include a part in which the diameter is substantially constant from one end to the other end of the portion (claim 2), the guide portion may include a part in which the diameter gradually decreases from one end toward the other end of the guide portion in the axial direction, and the guide portion may be integrated with the drill bar such that the other end side faces the blade portion (claim 3).

To attain the above-mentioned object, the dental implant jig set according to the present invention includes the plurality of dental implant jigs from any of the first to third aspects of the present invention, wherein the dental implant jigs being different from each other in at least one of the largest diameter, the axial length, and the shape of the guide portion (claim 4).

To attain the above-mentioned object, the drill bar according to the present invention is integrated with the dental implant jig from any of the first to third aspects of the present invention by attachment or integrated molding (claim 5).

To attain the above-mentioned object, the drill bar set according to the present invention includes the plurality of drill bars according to claim 5, the drill bars being different from each other in at least one of the largest diameter, the axial length, and the shape of the guide portion, and the distance from the front end of the blade portion to the guide portion (claim 6).

### EFFECTS OF THE INVENTION

The present invention provides a dental implant jig, a dental implant jig set, a drill bar, and a drill bar set, which can safely and easily achieve accurate drilling at low costs.

That is, at drilling of a fixture embedding hole used, for example, for implant prosthesis of partial chipping, the operator creates an image of the ideal embedding hole and an image of an upper prosthesis to be attached, and decides the position and the direction (a starting point and an embedding angle) of drilling on the basis of the images. Then, the jig according to the present invention that functions as a stent integrated with a drill bar is useful for such decision. That is, the jig rotates around the center of the shaft together with the shaft, and at least at this rotation, forms a guide portion that is concentric with the shaft and has a larger diameter than the shaft. The guide portion is made closer to the upper prosthesis to be attached in size and shape than the shaft, which improves the correctness of the images envisaged by the operator in deciding the position and the direction of drilling. Moreover, the guide portion is located closer to the next tooth at drilling than the shaft, and the closeness to the next tooth restricts shaking of the shaft at drilling. Accordingly, the process from creation of the images to decision of the position and the direction of drilling, in turn, drilling itself can be performed accurately and easily by using the jig.

Moreover, since the jig of the present invention rotates with the drill bar, there is no possibility that a part of the jig is cut with the drill bar and enters into the embedding hole, which is safe. The jig of the present invention also functions as a depth-adjusting stopper for preventing excessive drilling.

Further, by using the jig having a diameter (largest diameter) that is equivalent (equal) to the largest outer diameter of an impression coping at impression after implant embedding (or the drill bar integrated with the jig), the impression coping can be reliably prevented from contacting the tooth.

The jig from the first to fourth aspect of the present invention performs its function merely by being attached to the drill bar, can be easily attached to/detached from the drill bar without any complicated technique, and can be made of silicone rubber or the like at low costs.

Especially, from the third aspect of the present invention, by forming a desired tapered angle on the outer circumferential surface of the guide portion, tilted implantation at an intended angle can be easily achieved.

### BRIEF DESCRIPTION OF THE DRAWINGS

Fig. 1 is a view schematically showing a drilling method using a dental implant jig in accordance with an embodiment of the present invention.
Fig. 2 is a view schematically showing a conventional drilling method.
Figs. 3(A) to 3(C) are a plan view, a perspective view, and a front view, which schematically show the dental implant jig, respectively.
Fig. 4 is a view schematically showing a drilling method using a dental implant jig in a modification example of the present invention.
Fig. 5 is a view schematically showing a configuration of the dental implant jig in Fig. 4.
Figs. 6(A) and 6(B) are a perspective view and a front view, which schematically show a configuration of a 30 degree-dental implant jig, respectively.
Figs. 7(A) and 7(B) are a perspective view and a front view, which schematically show a configuration of a 45 degree-dental implant jig, respectively.
Fig. 8 is a view schematically showing a configuration of a roll-like dental implant jig in a modification example of the present invention.
Fig. 9 is a view schematically showing a configuration of a substantially C-like dental implant jig in a modification example of the present invention.
Fig. 10 is a perspective view schematically showing a configuration of a dental implant jig having a straight portion and a narrowed portion in a modification example of the present invention.
Fig. 11(A) is a front view schematically showing a configuration of a dental implant jig having guiding tapered portion in a modification example of the present invention, and Fig. 11(B) is a sectional view schematically showing a configuration of the dental implant jig having a stepped portion on the inner circumferential surface of a hollow shaft central portion.
Fig. 12 (A) and 12 (B) are perspective views schematically showing an example and another example of a drill bar in a modification example of the present invention.
Fig. 13 is a perspective view schematically showing a dental implant jig set in accordance with another embodiment of the present invention.
Fig. 14 is a perspective view schematically showing a drill bar set in accordance with another embodiment of the present invention.

### DESCRIPTION OF REFERENCE SIGNS

1 dental hand piece
2 drill bar
2a shaft
2b blade portion
5 dental implant jig
M guide portion

### DESCRIPTION OF THE EMBODIMENT

An embodiment of the present invention will be described below with reference to drawings.

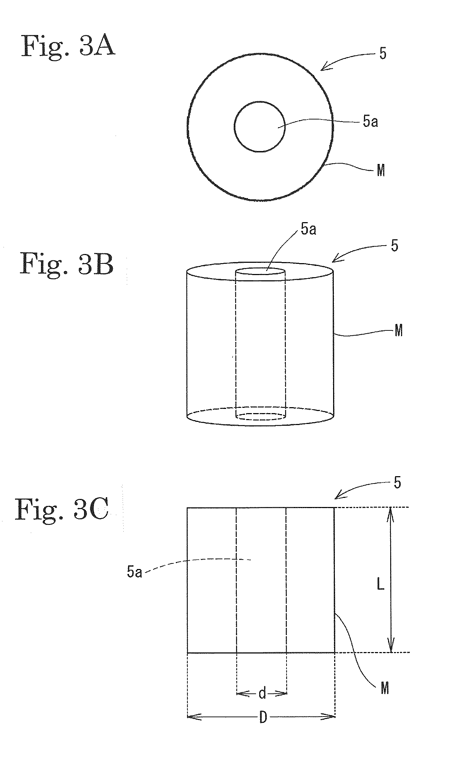

As shown in Fig. 1 and Figs. 3(A) to 3(C), a dental implant jig in accordance with this embodiment (hereinafter abbreviated as "jig") 5 is a substantially cylindrical guide (sleeve) member, and is provided at a drill bar (dental bar) 2 of a dental hand piece 1 to act as a guide for the position and/or the direction of drilling for forming an implant fixture embedding hole. Both of the dental hand piece 1 and the drill bar 2 may be commercialized products (general-purpose products).

The drill bar 2 has an attachment portion (not shown) attached to the hand piece 1 at one end and a blade portion 2b at the other end. The shape and the dimension of the blade portion 2b vary depending on the type (round bar, twist bar, and so on) and the manufacturer of the drill bar 2, and the drill bar 2 that includes the shaft 2a having a diameter of about 2 mm and is interposed between the attachment portion and the blade portion 2b as described above, is often adopted.

The jig 5 has a hollow shaft central portion 5a into which the shaft 2a is passed and press-fitted. The jig 5 in this embodiment has a diameter D of 6 mm (Refer to Fig. 3(C)) and an axial length L of 6mm (Refer to Fig. 3(C)) and however, the diameter D and the length L may be appropriately changed according to the case. For example, the diameter D may be in the range of 5 to 12 mm (preferably, 5 to 9 mm), and the length L may be in the range of 3 to 15 mm.

In this embodiment, the jig 5 is formed of an elastic body (rubber elastic body or the like) to be externally press-fitted to the shaft 2a, and the hollow shaft central portion 5a is a through hole having an inner diameter d slightly smaller than the outer diameter of the shaft 2a (Refer to Fig. 3(C)). In this embodiment, the inner diameter d is set to 2mm.

Elastomers such as rubber (synthetic rubber and natural rubber) and resins (synthetic resin and natural resin), and sponges may be used as materials for the jig 5, and examples of the materials include silicone rubber, urethane rubber, fluorine rubber, chloroprene rubber, nitrile-butadiene rubber, ethylene-propylene rubber, styrene-butadiene rubber, olefin rubber, fluorine resin, polyethylene resin (high to low densities), polypropylene resin, silicone rubber sponge, and fluorine rubber sponge. However, in terms of heat resistance to heat sterilization, easy availability, cost efficiency, electrical insulation property, biocomparability, chemical resistance, weather resistance, and long-term storage performance, silicone rubber is the most preferable at the present time.

The jig 5 may be externally press-fitted to the drill bar 2 either from one end (the side of the attachment portion to the hand piece 1) or the other end (the side of the blade portion 2b). The jig 5 at drilling (during use) may cover only the shaft 2a, or may cover a part (upper part) of the blade portion 2b as well.

For example, at drilling of the fixture embedding hole used for implant prosthesis of partial chipping, the operator creates the image H of the ideal embedding hole (Refer to Fig. 1) and the image of the upper prosthesis to be attached, and decides the position and the direction of drilling (starting point and embedding angle) on the basis of the images. The jig 5 that functions as a stent integrated with the drill bar 2 is useful for such decision. That is, when the jig 5 is attached to the drill bar 2, the jig 5 integrated with the shaft 2a rotates around the center of the shaft 2a together with the shaft 2a, and the outer circumferential surface of the jig 5 becomes a tubular guide portion M that is concentric with the shaft 2a and has a larger diameter than the shaft 2a. The guide portion M has a size and shape closer to the upper prosthesis to be attached than that of the shaft 2a, improving the correctness of the images envisaged by the operator in deciding the position and the direction of drilling. Moreover, the guide portion M is located closer to the next tooth at drilling than the shaft 2a, and the closeness to the next tooth restricts shaking of the shaft 2a at drilling. Accordingly, the process from creation of the image H (image formation) to decision of the position and the direction of drilling, in turn, drilling itself can be performed accurately and easily by using the jig 5.

By forming a bone hole of a certain depth (for example, bone hole having an substantially half depth of a predetermined depth of the fixture embedding hole) by drilling using the jig 5, the bone hole itself can act to guide drilling up to the predetermined depth. Thus, when the bone hole thus formed starts to perform the guiding function, the jig 5 may be detached from the drill bar 2.

Since the jig 5 attached to the drill bar 2 rotates with the drill bar 2, there is no possibility that a part of the jig 5 is cut with the drill bar 2 and enters into the embedding hole, which is safe. Further, the jig performs its function merely by being attached to the drill bar 2, can be easily attached to/detached from the drill bar without any complicated technique, and can be made of silicone rubber or the like at low costs. The jig 5 can be used as a depth-adjusting stopper for preventing excessive drilling.

The jig 5 is suitable for drilling for implant prosthesis of partial chipping as shown in Fig. 1 and however, the present invention is not limited to this, and can be also applied to implant prosthesis of teeth chipping and free end chipping.

An experiment for considering the effectiveness of the jig 5 will be described below. In this experiment, each estimator (operator) formed an implant embedding cavity in a model twice, and measured the embedding angle and the starting point of drilling each time. Five dentists (n = 5) who had looked on an implant surgery, but had not actually embedded an implant in clinical and model trainings were selected as the estimators. Each estimator formed the implant embedding cavity twice: a case of freehand without using the jig 5 (hereinafter referred to as "freehand case") ; and a case where the jig 5 was attached to the drill bar 2 (hereinafter referred to as "jig-using case").

A twist drill having a diameter of 2.0 mm was used as the drill bar 2, and in the jig-using case, the silicone rubber jig 5 having a hardness of 50 (and the same shape and size as described above) was attached to the drill bar 2.

A jaw model for training (E1 model manufactured by Nissin Dental Products INC.) used for the case of partial chipping of a left lower jaw first molar was used as a model for forming the implant cavity. A tooth axis of the jaw model having the left lower jaw first molar was measured, the tooth axis was set to an ideal implant axis, and an ideal starting point was set based on the tooth axis.

The embedding angle and the starting point of drilling were measured by using a digital camera, and three following programs: Photoshop (trademark) (ver.7.0, ADOBESYSTEMS, INC., U.S.), Microsoft Excel (trademark) (Microsoft Corporation, U.S.), and Stat View (trademark) (SAS Institute Inc., U.S.).

That is, the drill bar 2 inserted into the model by drilling was photographed with the digital camera from a predetermined position with reference to an occlusal surface, mesial and distal surfaces, and buccolingual surfaces under the same conditions. Then, using the above-mentioned three programs, data acquired by photographing was analyzed to find an angle error (deviation angle) relative to the ideal implant axis and a distance error (deviation distance) relative to the ideal starting point in the form of [(absolute) average value ± standard deviation (SD)] (n = 5), as results of drilling.

The angle error relative to the ideal implant axis in the mesial and distal direction was [6.84 ±3.67°] in the freehand case and [4.84 ± 2.80°] in the jig-using case, and the angle error in the buccolingual direction was [9.38 ± 3.48°] in the freehand case and [6.34 ± 6.10°] in the jig-using case.

The distance error relative to the ideal starting point in the mesial and distal direction was [1.24 ± 0.81 mm] in the freehand case and [0.16 ± 0.11mm] in the jig-using case, and the distance error in the buccolingual direction was [0.48 ± 0.49 mm] in the freehand case and [0.44 ± 0.10mm] in the jig-using case.

The above-mentioned results demonstrated that the jig-using case achieved more ideal drilling in terms of both of the embedding angle and the starting point in the mesial and distal direction and the buccolingual direction than the freehand case.

The present invention is not limited to the embodiment, and as a matter of course, may be variously modified so as not to deviate from the subject matter of the present invention. For example, following modification examples are possible.

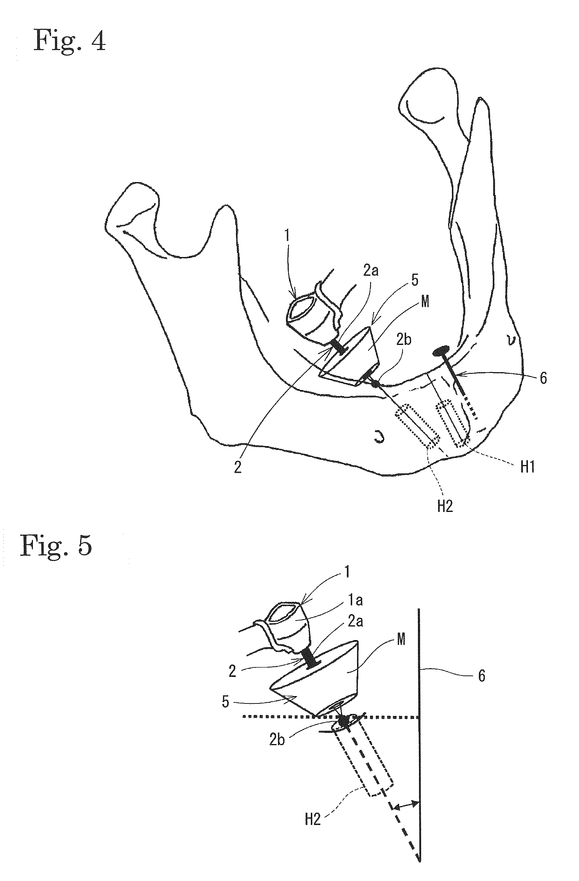

In the embodiment, the outer diameter (diameter) D of the jig 5 is substantially uniform from its one end to the other end and however, the present invention is not limited to this, and for example, as shown in Fig. 4 to Fig. 6, the outer diameter D of the jig 5 may be gradually decreased from the one end toward the other end in the axial direction, and the jig 5 may be attached to the drill bar 2 such that the other end side faces the blade portion 2b. The use of such jig 5 can simplify All on 4 (all on four), All on 5 (all on five), and All on 6 (all on six) with a tilted embedding implant (tilted implantation) used in, for example, complete denture-typed implant prosthesis.

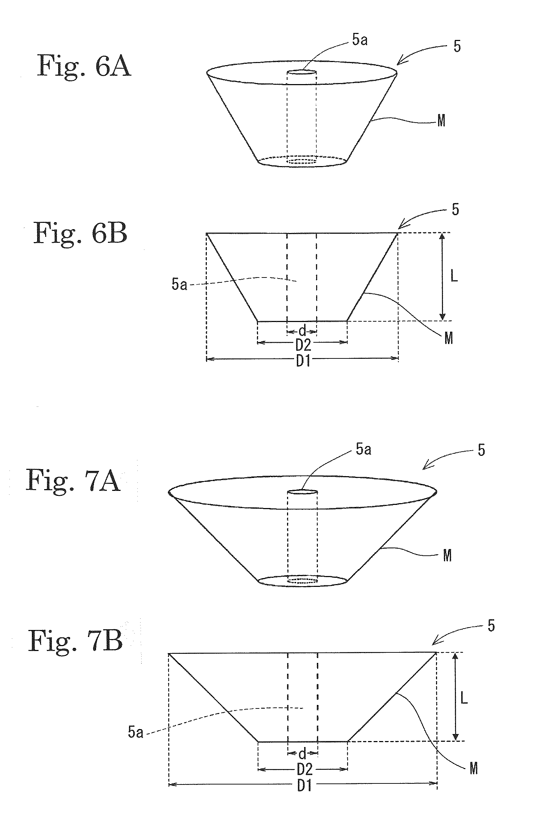

Describing a method of performing All on 4 (5, 6) with the jig 5 in detail with reference to Fig. 4, first, a guide pin 6 having a diameter of 2 mm, for example, is vertically driven into a jaw bone (upper jaw or lower jaw). Then, in the case of a long-axis (vertical) embedding implant (axial implantation), by attaching the jig 5 shown in Figs. 3(A) to 3(C) to the drill bar 2, and performing drilling such that the straight outer circumferential surface of the jig 5 becomes parallel to the guide pin 6, a fixture embedding hole H1 vertical to the jaw bone (Refer to Fig. 4) can be easily formed. In the case of the tilted embedding implant, by attaching the jig 5 shown in Figs. 6 (A) and 6(B) to the drill bar 2 and performing drilling such that the tapered outer circumferential surface of the jig 5 becomes parallel to the guide pin 6 (Refer to Fig. 5), the fixture embedding hole H2 tilted relative to the jaw bone by a predetermined angle without passing nerves, blood vessels, and maxillary sinus (Refer to Fig. 4) can be easily formed. After formation of the embedding holes H1 and H2, the guide pin 6 is removed.

In the case of the tilted embedding implant as described above, the tilted angle can be freely set by changing the outer circumferential surface of the tapered angle of the jig 5, and for example, the tapered angle may be selected in the range of 45 degrees or less. Figs. 6 (A) and 6 (B) show the 30 degree-jig 5 having a tapered angle of 30 degrees, the outer diameter D1 at one end is 13 mm, the outer diameter D2 at the other end is 6 mm, and the axial length L is 6 mm. Figs. 7 (A) and 7 (B) show the 45 degree-jig 5 having a tapered angle of 45 degrees, the outer diameter D1 at one end is 18 mm, the outer diameter D2 at the other end is 6 mm, and the axial length L is 6 mm.

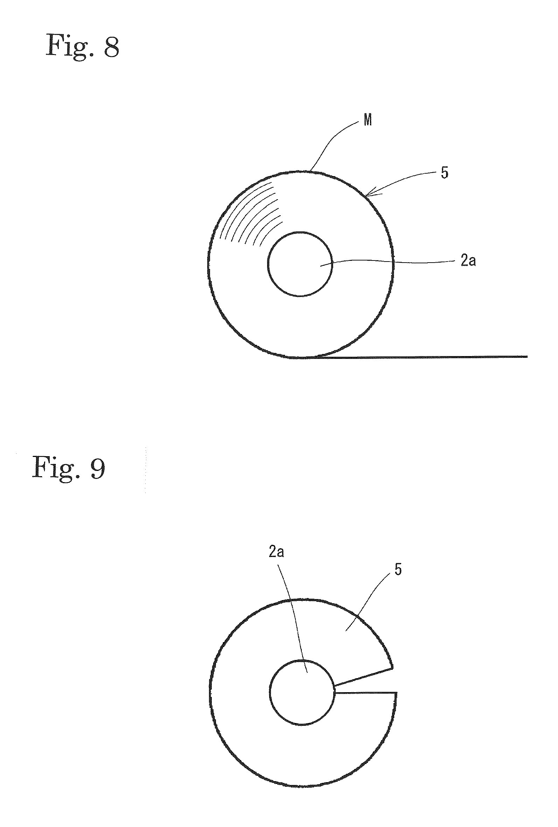

In the embodiment, the substantially tubular jig 5 is made of an elastic material and however, the present invention is not limited to this, and the jig 5 may be configured by winding a paper sheet-like member into a roll and adhering at least its ends thereto (Refer to Fig. 8). In this case, the outer diameter D of the jig 5 may be decreased by sequentially unreeling the sheet-like member wound into the roll from the outermost portion to decrease the number of windings of the roll, or may be increased by providing a portion unwound into the roll on the sheet-like member and winding the unwound portion onto the outside of the previously-wound portion. The sheet-like member constituting the jig 5 may be wound from the unfolded state without being initially wound into the roll. When, in the sheet-like member, a portion unwound into the roll is generated, the unwound portion may be cut with any proper means such as scissors.

The jig 5 may be made of metals such as stainless, iron, and titanium, and carbon. That is, for example, the jig 5 may be a metal member that can move in the axial direction of the shaft 2a, and the movement of the jig 5 may be restricted by attaching a stopper member (not shown) made of an elastic material to the shaft 2a.

In the embodiment, the jig 5 has an annular shape (substantially cylindrical shape) in which a cut surface orthogonal to the center of the shaft is concentric with the center of the shaft and however, the present invention is not limited to this, and the jig 5 may have an arcuate and substantially C-like shape (columnar shape having a substantially C-like horizontal surface) (Refer to Fig. 9). In this case, since the hollow shaft central portion 5a is not a through hole, but is a notched groove communicating with the outer circumference of the jig 5, the jig 5 can be directly press-fitted to the shaft 2a from the circumference without sliding the jig 5 toward the shaft 2a, after externally fitting the jig 5 from one of both ends of the drill bar 2.

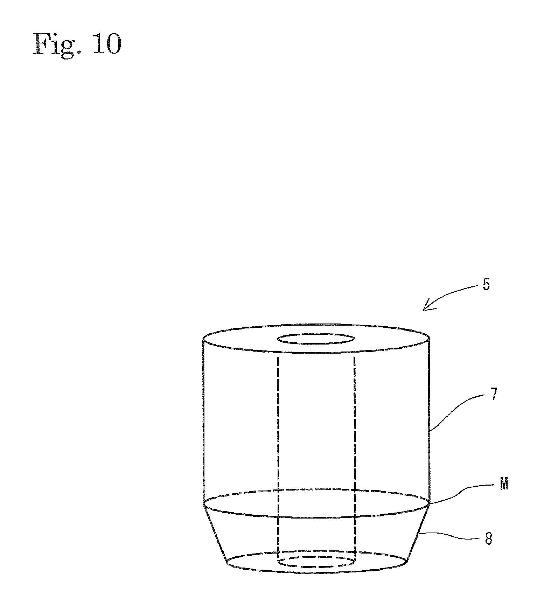

Fig. 3 shows the case where the outer diameter D is substantially constant from one end to the other end of the jig 5 in the axial direction, and Fig. 6 and Fig. 7 show the case where the outer diameter D is tapered at a certain rate from the one end toward the other end of the jig 5 in the axial direction. However, the outer diameter D of the jig 5 may irregularly vary in the axial direction. That is, the jig 5 may be formed by combining a straight portion in which the outer diameter D is substantially constant from its one end to the other end, a narrowed portion in which the outer diameter D decreases at a regular or irregular rate from the one end toward the other end, and an extended portion in which the outer diameter D increases at a regular or irregular rate from the one end toward the other end, in a various way. As an example, Fig. 10 shows the jig 5 including a straight portion 7 and a narrowed portion 8.

In the case where the cut surface orthogonal to the center of the shaft is annular and is concentric with the center of the shaft as in the jig 5 shown in Fig. 3, Fig. 6, Fig. 7, and Fig. 10, the outer circumferential surface of the jig 5 becomes the guide portion M that is concentric with the shaft 2a and has a larger diameter than the shaft 2a, and excites supports for decision of the position and the direction of drilling both during rotation and rest of the jig 5.

However, even in the case where the cut surface orthogonal to the center of the shaft is not annular and is not concentric with the center of the shaft as in the jig 5 shown in Fig. 9, when the shaft 2a rotates at a rotation rate at which the dental hand piece 1 applies to the drill bar 2, and if the jig 5 can form the guide portion M that is concentric with the shaft 2a and has a larger diameter than the shaft 2a on the outer side of the shaft 2a by an after-image phenomenon, the jig 5 can perform the above-mentioned supporting function. Accordingly, to the extent that the jig 5 performs the supporting function, the jig 5 can be variously deformed, for example, various grooves or protrusions may be provided on the outer circumference of the jig 5, or the outer circumferential surface of the jig 5 may be shaped to have a polygonal horizontal surface.

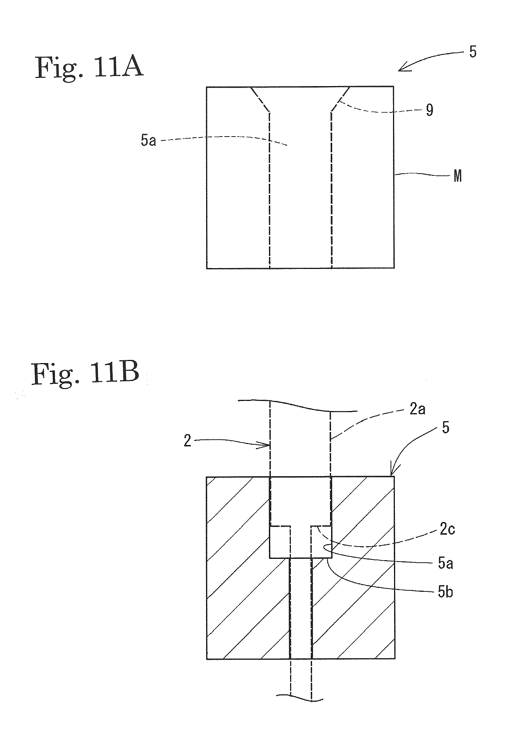

For example, a plurality of vertical grooves (axial grooves) or horizontal grooves (circumferential grooves) are provided in the inner circumferential surface of the hollow shaft central portion 5a shaped as the through hole to reduce the force necessary for externally press-fitting the jig 5 to the shaft 2a. As shown in Fig. 11(A), a guiding tapered portion 9 for guiding external press-fitting of the jig 5 to the drill bar 2 may be provided at an end of the inner circumferential surface of the hollow shaft central portion 5a, and the guiding tapered portion 9 may be provided at each end of the inner circumferential surface. For example, as shown in Fig. 11(B), in the case where an outer-diameter changed portion (stepped portion in the illustrated example) 2c, the outer diameter of which changes, is present in the shaft 2a of the drill bar 2, an inner-diameter changed portion (stepped portion in the illustrated example) 5b, like the outer-diameter changed portion 2c, the inner diameter of which changes, may be provided in the inner circumferential surface of the hollow shaft central portion 5a of the jig 5, such that the jig 5 can be suitably attached so as to cover the outer-diameter changed portion 2c of the shaft 2a. In this case, the position of the inner-diameter changed portion 5b in the hollow shaft central portion 5a can be appropriately decided. The outer-diameter changed portion 2c and the inner-diameter changed portion 5b each are not limited to the stepped portion, and may be a tapered portion.

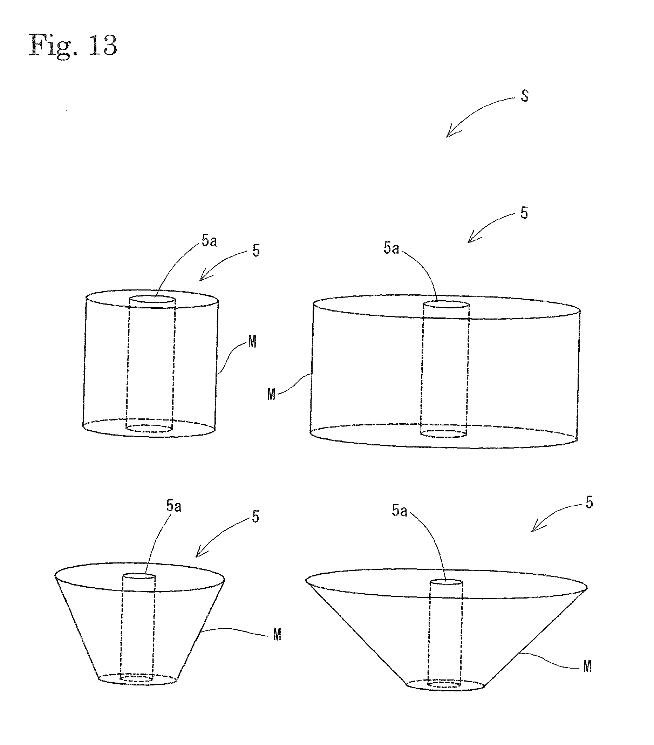

The diameter D (largest diameter), the axial length L, and the shape of the guide portion M (jig 5) may be adjusted according to the case. For this reason, a plurality of jigs 5 that are different from each other in at least one of the diameter D (largest diameter), the axial length L, and the shape of the guide portion M (jig 5) may be prepared as a set (jig set S) (Refer to Fig. 13), and the appropriate jig 5 may be selected from the plurality of jigs 5. In this case, the color and the shape of the jig 5 may be varied according to size such that the size of the jig 5 (guide portion M) can be recognized at a glance. To this end, for example, one or more vertical grooves (axial grooves) or horizontal grooves (circumferential grooves) may be provided in the outer circumferential surface of the jig 5, and the number of the grooves or interval between the grooves may be varied.

By using the jig 5 having a diameter D (largest diameter) that is equivalent (equal) to the largest outer diameter of an impression coping at impression after drilling, the impression coping can be reliably prevented from contacting the tooth.

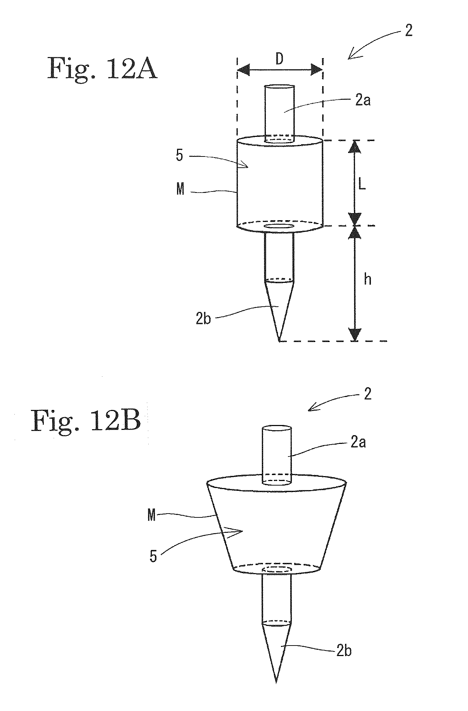

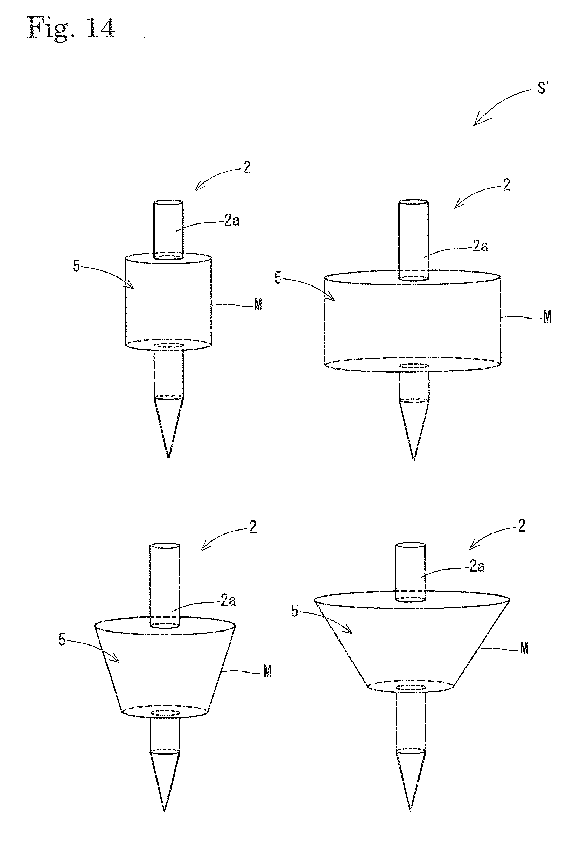

In the embodiment, the drill bar 2 is integrated with the separate jig 5 by attachment, but may be integrated by molding. Figs. 12(A) and 12(B) show an example and another example of the drill bar 2 integrated with the jig 5, the jig 5 shown Fig. 12 (A) is the same as that shown in Fig. 3, and the jig 5 shown in Fig. 12 (B) is the same as that shown in Fig. 6 in shape. As a matter of course, in the drill bar 2 integrated with the jig, the largest diameter (diameter D), the axial length L, and the shape of the guide portion M (jig 5) can be variously modified. For the drill bar 2, a distance (depth) h between the front end of the blade portion 2b and the guide portion M (jig 5) (Refer to Fig. 12(A)) can be also variously modified. Thus, a plurality of drill bars 2 that are different from each other in at least one of the largest diameter (diameter D), the axial length L, the shape, and the depth h of the guide portion M (jig 5) may be prepared as a set (drill bar set S') (Refer to Fig. 14), and the appropriate drill bar 2 may be selected from the set. The drill bar 2 including no jig 5 may be included in the drill bar set, and for example, when a bone hole of certain size is created by drilling, the drill bar 2 including no jig 5 may be used.

It is needless to say that the modification examples may be combined with each other.

### INDUSTRIAL APPLICABILITY

A dental implant jig, a dental implant jig set, a drill bar and a drill bar set according to the present invention can be used for a drilling operation in dental implant treatment.

## Claims

1. A dental implant jig **characterized by** being provided at a drill bar of a dental hand piece to act as a guide in position and/or direction of drilling for forming an implant fixture embedding hole, being integrated with a shaft interposed between an attachment portion on one side of the drill bar, which is attached to the hand piece, and a blade portion on the other side of the drill bar, rotating around the center of the shaft together with the shaft, and forming a guide portion that is provided on the outer side of the shaft concentrically with the shaft and has a larger diameter than the shaft.

2. The dental implant jig according to claim 1, wherein the guide portion includes a part in which the diameter is substantially constant from one end to the other end of the portion.

3. The dental implant jig according to claim 1, wherein the guide portion includes a part in which the diameter gradually decreases from one end toward the other end of the guide portion in the axial direction, and the guide portion is integrated with the drill bar such that the other end side faces the blade portion.

4. A dental implant jig set **characterized by** including the plurality of dental implant jigs according to anyone of claims 1 to 3, wherein the dental implant jigs being different from each other in at least one of the largest diameter, the axial length, and the shape of the guide portion.

5. A drill bar **characterized by** being integrated with the dental implant jig according to anyone of claims 1 to 3 by attachment or integrated molding.

6. A drill bar set **characterized by** including the plurality of drill bars according to claim 5, the drill bars being different from each other in at least one of the largest diameter, the axial length, and the shape of the guide portion, and the distance from the front end of the blade portion to the guide portion.
